# EUROPEAN PATENT APPLICATION

(11) **EP 1 738 643 A1**
(43) Date of publication of application: **03.01.2007**
(21) Application number: 05730565.8
(22) Date of filing: 15.04.2005
(51) Int. Cl.: A01K 67/027, C12N 15/09, C12Q 1/02, G01N 33/15, G01N 33/50, G01N 33/53

(54) **TLR LIGAND AND IL-1 RESPONSE-INJURED ANIMAL MODEL**

(30) Priority: 20.04.2004 JP 2004124758
(71) Applicant: Japan Science and Technology Agency, Kawaguchi-shi, Saitama 332-0012 (JP)
(72) Inventor: AKIRA, Shizuo, Osaka 569-0036 (JP); YAMAMOTO, Masahiro, Osaka 562-0031 (JP)
(74) Representative: Harding, Charles Thomas
(86) International application number: PCT/JP2005/007304
(87) International publication number: WO 2005/102040

(57) **Abstract**

The present invention provides a TLR ligand/IL-1 response-impaired non-human animal model wherein IL-6 production responding to a TLR ligand/IL-1 is impaired, a method for screening a substance promoting or suppressing a response to a TLR ligand/IL-1 by using the TLR ligand/IL-1 response-impaired non-human animal model, and a method for screening a prophylactic/therapeutic agent for atopic dermatitis-like inflammatory skin disease. A non-human animal model unresponsive to a TLR ligand/IL-1 is generated wherein whole or a part of an endogenous gene of the non-human animal encoding IκB-ζ is inactivated by a gene mutation such as destruction, deletion, or substitution, a function of expressing IκB-ζ is deleted, and a IL-6 production responding to a TLR ligand and IL-1 is impaired. Moreover, the promoter or suppressor for a TLR ligand/IL-1 response and the prophylactic/therapeutic agent for atopic dermatitis-like inflammatory skin disease are screened by using the non-human animal and a test substance.

SEQUENCE LISTING

<110>  Japan Science and Technology Agency

<120>  TLR Ligand and IL-1 Response-Injured Animal Model

<130>  P027270EP

<140>  05730565.8
<141>  2005-04-15

<150>  JP2004-124758
<151>  2004-04-20

<160>  6

<170>  PatentIn version 3.1

<210>  1
<211>  27
<212>  DNA
<213>  Artificial

<220>
<223>  Primer for kB Region of IL-6 Promoter

<400>  1
cgatgctaaa cgacgtcaca ttgtgca                                          27

<210>  2
<211>  27
<212>  DNA
<213>  Artificial

<220>
<223>  Primer for kB Region of IL-6 Promoter

<400>  2
ctccagagca gaatgagcta cagacat                                          27

<210>  3
<211>  27
<212>  DNA
<213>  Artificial

<220>
<223>  Primer for 3' Gene Segment of IL-6 Promoter

<400>  3
gcagatggac ttagctcgtc tcattca                                          27

<210>  4
<211>  27
<212>  DNA
<213>  Artificial

<220>
<223>  Primer for 3' Gene Segment of IL-6 Promoter

<400>  4
ccactccttc tgtgactcca gcttatc                                          27

<210>  5
<211>  27
<212>  DNA
<213>  Artificial

<220>
<223>  Primer for kB Region of ELAM 1 Promoter

<400>  5
gatgcagttg agaatttcct cttagcc                                          27


<210>  6
<211>  27
<212>  DNA
<213>  Artificial

<220>
<223>   Primer for kB Region of ELAM 1 Promoter

<400>  6
tggaatagtt gttctggcgt tggatcc                                          27

## Description

### Technical Field

The present invention relates to a Toll-like receptors (TLR) ligand and Interleukin-1 (IL-1) response-impaired non-human animal model wherein an IκB-ζ gene has been knocked-out, and a method for screening a substance promoting or suppressing a response to a TLR ligand or IL-1 by using the non-human animal model.

### Background Art

The innate immune system is a biological defense system responsible for sensing and eliminating invasion of pathogens such as bacteria and viruses. Once the innate immune system detects the presence of microorganisms, inflammatory cytokines are produced, inducing inflammations in vivo. At the same time, activation of lymphocytes such as T and B cells is induced to establish an infection-defense mechanism against invaded microorganisms. TLR family is known as receptors involved in the recognition of microorganisms in the innate immune system. It is known that stimulation of TLR family and pro-inflammatory interleukin-1 receptor (IL-1R) family members, which have intracellular regions homologous to intracellular domains of TLR family, activates transcription factors NF-κB, AP-1, and the like, and culminates in induction of expression of many proteins including inflammatory cytokines such as TNF-α, IL-6, and IL-12p40.

One of genes relating to the immune responses includes a nuclear protein called IκB-ζ (also known as MAIL and INAP) (see, for example, non-patent documents 1-6). IκB-ζ is a nuclear protein that carries ankyrin repeats in its C-terminal portion, and shows high homology with IκB family member, Bcl-3 (see, for example, non-patent documents 4-6). *In vitro* studies have shown that ectopic expression of IκB-ζ inhibits activities of the NF-κB-responsive reporter, suggesting that IκB-ζ acts as a negative regulator for NF-κB (see, for example, non-patent document 4). On the other hand, another study shows that IκB-ζ overexpression promotes LPS-induced IL-6 production in fibroblasts, indicating positive effects of IκB-ζ on the IL-6 production (see, for example, non-patent document 5). Thus, whether IκB-ζ functions as a potent co-activator or co-repressor remains to be seen. Stimulation with IL-1 or LPS has been shown to induce IκB-ζ in macrophage cell lines and fibroblasts. It has also been known that IL-1R and the receptor for LPS, TLR4, share intracellular signaling pathways (see, for example, non-patent documents 1-3).

TLR and IL-1R signaling pathways utilize an adaptor protein, MyD88, to signal downstream, resulting in expression of inflammatory mediators including Cox-2 (see, for example, non-patent documents 7-9). In addition to the MyD-88-dependent pathway, there is a MyD88-independent pathway in TLR3 and TLR4 signaling pathways. The MyD88-independent pathway induces the expression of type I interferon (IFN) and IFN-inducible genes such as IP-10 (see, for example, non-patent documents 10-12). The TLR/IL-1R-mediated MyD88-dependent pathways for the inflammatory cytokine production is known to activate NF-κB and mitogen-activated protein(MAP) kinases (see, for example, non-patent documents 13-15).

Non-patent document 1: Annu. Rev. Immunol . 21, 335-376 (2003)
Non-patent document 2: Annu. Rev. Immunol. 20, 197-216 (2002)
Non-patent document 3: Curr Opin Pharmacol. 3, 396-403 (2003),
Non-patent document 4: J Biol Chem. 276, 27657-27662 (2001)
Non-patent document 5: FEBS Lett. 485, 53-56 (2000)
Non-patent document 6: J Biol Chem. 276, 12485-12488 (2001)
Non-patent document 7: Science. 278, 1612-1615 (1997)
Non-patent document 8: Immunity. 7, 837-847 (1997)
Non-patent document 9: Mol Cell. 2, 253-258 (1998)
Non-patent document 10: Nature. 413, 732-738 (2001)
Non-patent document 11: J Immunol. 167, 5887-5894 (2001)
Non-patent document 12: Immunity. 17,251-63 (2002)
Non-patent document 13: Cell. 109 Suppl, S81-96 (2002)
Non-patent document 14: J Endotoxin Res. 6, 453-457 (2000)
Non-patent document 15: Mol Cell. 11, 293-302 (2003)

### Disclosure of the Invention

### Object to be solved by the present invention

The object of the present invention is to provide a TLR ligand and IL-1 response-impaired non-human animal model wherein IL-6 production responding to a TLR ligand and IL-1 is impaired. The object of the present invention is also to provide a method for screening a substance promoting or suppressing a response to a TLR ligand or IL-1 by using the TLR ligand and IL-1 response-impaired non-human animal model, and a method for screening a prophylactic/therapeutic agent for atopic dermatitis-like inflammatory skin disease.

### Means to solve the object

By generating a mouse lacking IκB-ζ, which is a protein induced by an activation of TLR or IL-1R signal, expression of a certain type of genes, such as IL-6 and IL-12p40 was almost not observed by a stimulation of TLR or IL-1R ligand (components binding to a receptor). Therefore, it was found that IκB-ζ was indispensable for the expression of a group of genes activated in TLR/IL-1R signaling pathways. Furthermore, it was found that, even when TNF-α production is normal, IκB-ζ-deficient mouse showed severe impairment in the IL-6 production in response to a TLR ligand and IL-1, that IκB-ζ overexpression up-regulates basal activities of the IL-6 promoter via transcription factor NF-κB, and also that endogenous IκB-ζ specifically associates with a p50 subunit of the NF-κB and is recruited to a proximal region of the IL-6 promoter upon stimulation. Moreover, it was found that NF-κB1/p50-deficient mice show similar phenotypes to IκB-ζ-deficlent mice in terms of the TLR/IL-1R-mediated responses, and that endotoxin-induced expression of other genes such as IL-12p40 and GM-CSF is abrogated in IκB-ζ-deficient macrophages. Given that the LPS induction of IκB-ζ occurs earlier than that of IL-6, it is believed that some TLR/IL-1R-meidated responses may be regulated in at least a two-step gene expression that requires inducible IκB-ζ. The invention has completed based on the findings described above.

The present invention relates to: (1) a non-human animal model unresponsive to a TLR ligand and IL-1, wherein whole or a part of an endogenous gene of the non-human animal encoding IκB-ζ is inactivated by a gene mutation such as destruction, deletion, or substitution, a function of expressing IκB-ζ is deleted, and a IL-6 production responding to a TLR ligand and IL-1 is impaired; (2) the non-human animal model unresponsive to a TLR ligand and IL-1 according to (1), wherein the TLR ligand is LPS, BLP, PGN, MALP-2, R-848, or CpG DNA; (3) the non-human animal model unresponsive to a TLR ligand and IL-1 according to (1) or (2), wherein the non-human animal model shows an atopic dermatitis-like inflammatory skin disease; and (4) the non-human animal model unresponsive to a TLR ligand and IL-1 according to any one of (1) to (3), wherein the non-human animal is a mouse.

The present invention also relates to: (5) a method for screening a substance promoting or suppressing a response to a TLR ligand or IL-1, or a material containing the same, wherein the non-human animal according to any one of (1) to (4), a test substance, and a TLR ligand or IL-1, or a material containing the same are used to measure/evaluate an IL-6 production level responding to a TLR ligand or IL-1, or a material containing the same, in the non-human animal; and (6) a method for screening a substance promoting or suppressing a response to a TLR ligand or IL-1, or a material containing the same, wherein an immune cell derived from the non-human animal according to any one of (1) to (4), a test substance, and a TLR ligand or IL-1, or a material containing the same, are used to measure/evaluate an IL-6 production level responding to a TLR ligand or IL-1, or a material containing the same in the immune cell.

The present invention further relates to: (7) a method for screening a prophylactic/therapeutic agent for atopic dermatitis-like inflammatory skin disease, wherein a test substance is administrated to the non-human animal according to any one of (1) to (4) and a level of atopic dermatitis-like inflammatory skin disease in the non-human animal is measured/evaluated.

### Brief Description of Figures

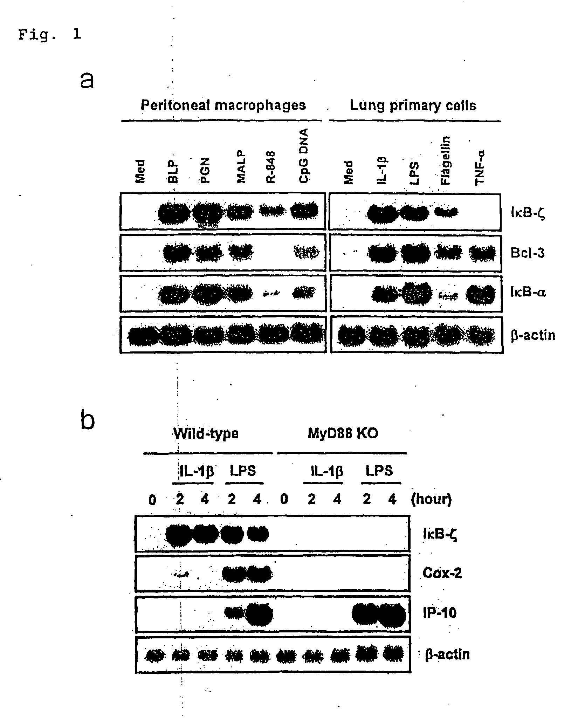

[Fig. 1] It is a figure showing that IκB-ζ is specifically induced upon TLR/IL-1R stimulation. a. Peritoneal macrophages and lung primary cells were stimulated with 100 ng ml⁻¹ BLP, 10 µg ml⁻¹ PGN, 30 ng ml⁻¹ MALP-2, 100 nM R-848, 3 µM CpG DNA, 10 ng ml⁻¹ IL-1β, 10 µg ml⁻¹ LPS, 100 ng ml⁻¹ flagellin, and 10 ng ml⁻¹ TNF-α for 2 hours. Total RNA (10 µg) was extracted and subjected to Northern blot analysis for investigating the expression of IκB-ζ, Bcl-3, IκB-α, and β-actin. b. MEFs from wild-type and MyD88-deficient mice were stimulated with 10 ng ml⁻¹ IL-1β and 10 µg ml⁻¹ LPS for the indicated periods. Total RNA (10 µg) was extracted and subjected to Northern blot analysis for investigating the expression of IκB-ζ, Cox-2, IP-10, and β-actin.

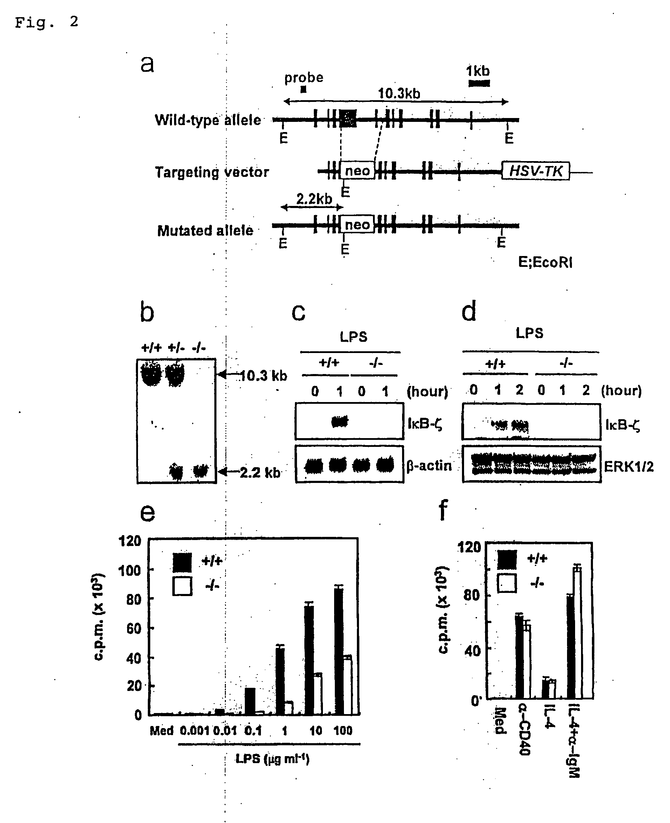

[Fig. 2] It is a figure showing target disruption of a mouse IκB-ζ gene. a. It is a figure showing the structure of an IκB-ζ gene, a targeting vector and a predicted disrupted gene. Black boxes denote an exon region to be IκB-ζ. Restriction enzymes: E, EcoRI. b. It is a figure showing a result of Southern blot analysis of an offspring mouse born from intercrossing heterozygotes. Genomic DNA was extracted from mouse tails, digested with EcoRI, electrophoresed, and hybridized with the radiolabelled probe indicated in a. Southern blotting gave a single 10.3-kb band for wild-type mice (+/+), a 2.2-kb band for homozygous mice (-/-) and both bands for heterozygous mice (+/-). c. It is a result of Northern blot analysis of peritoneal macrophages stimulated with 10 ng ml⁻¹ LPS. Total RNA (10 µg) extracted from cells was electrophoresed, transferred on a nylon membrane, and hybridized using IκB-ζ and β-actin. d. It is figure showing a result of Western blot analysis of peritoneal macrophages. The cell lysates were immunoblotted with α-IκB-ζ. The same lysates were also immunoblotted with α-ERK1/2 to monitor the protein expression. e, f. Splenocytes were cultured with the indicated concentration of LPS in the coexistence of 10 µg ml⁻¹ α-CD40, 200 U ml⁻¹ IL-4, and 10 µg ml⁻¹ α-IgM for 48 h. [³H]thymidine (1 µ Ci) was pulsed for the last 12 h of the culture. [³H]thymidine incorporation was measured by a scintillation counter (Packard).

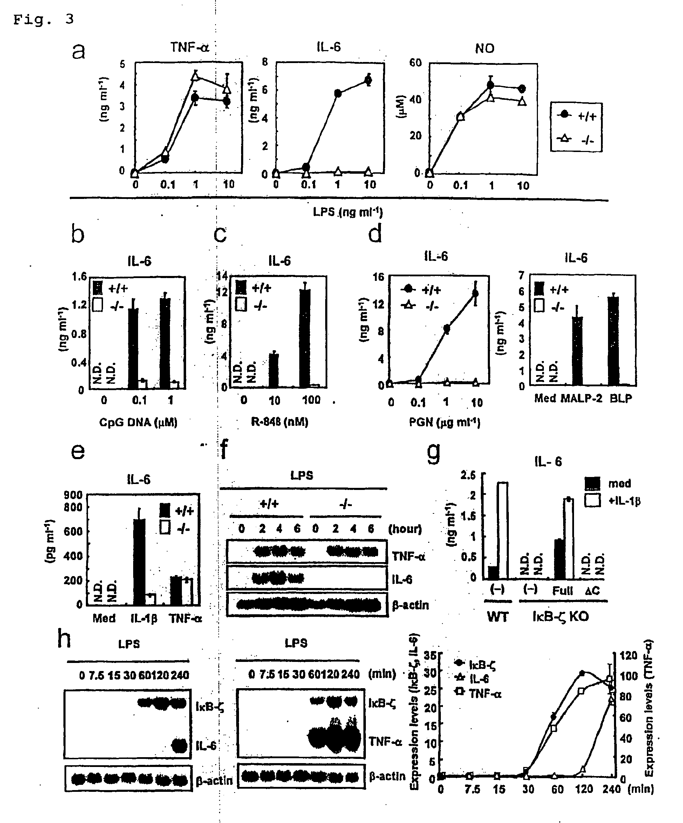

[Fig. 3] It is a figure showing immune responses in IκB-ζ-deficient cells, and kinetics of IκB-ζ induction. a, b, c, d. Peritoneal macrophages from wild-type and IκB-ζ-deficient mice were cultured with the indicated concentrations of PGN, R-848, CpG DNA in the coexistence of 10 ng ml⁻¹ LPS, 100 ng ml⁻¹ BLP, and 30 ng ml⁻¹ MALP-2, and in the presence of 30 ng ml⁻¹ IFN-γ for 24 h. Concentrations of IL-6, TNF-α, and NO in the culture supernatants were measured. The indicated values are means ± SD of triplicates. "N.D." means not detected. e. MEFs from wild-type and IκB-ζ-deficient mice were stimulated with 10 ng ml⁻¹ IL-1β and 10 ng ml⁻¹ TNF-α. Supernatents were collected for IL-6 analysis by ELISA 24 h later. Indicated values are means ± SD of triplicates. "N.D." means not detected. f. Peritoneal macrophages were stimulated with 10 ng ml⁻¹ LPS for the indicated periods. Total RNA (5 µg) was extracted and subjected to Northern blot analysis for investigating the expression of IL-6, TNF-α, and β-actin. g. It is a figure showing that IL-1 responsiveness in IκB-ζ-deficient MEFs is restored by retroviral transfection with full-length IκB-ζ (Full), but not by introduction of deletion mutant form IκB-ζ (ΔC) into cells. Concentrations of IL-6 in the culture supernatants, with or without 10 ng ml⁻¹ IL-β, were measured by ELISA. Indicated values are means ± SD of triplicates. h. It is a figure showing double RNA products indicative of mRNA transcripts derived from IκB-ζ, IL-6, and TNF-α following LPS simulation of wild-type peritoneal macrophages resolved by electrophoresis. Two-independent experiments with independently derived wild-type cells were quantitated by Phospholmager and mRNA amount was graphed for the indicated genes in arbitrary units (left for IκB-ζ and IL-6, right for TNF-α) relative to β-actin.

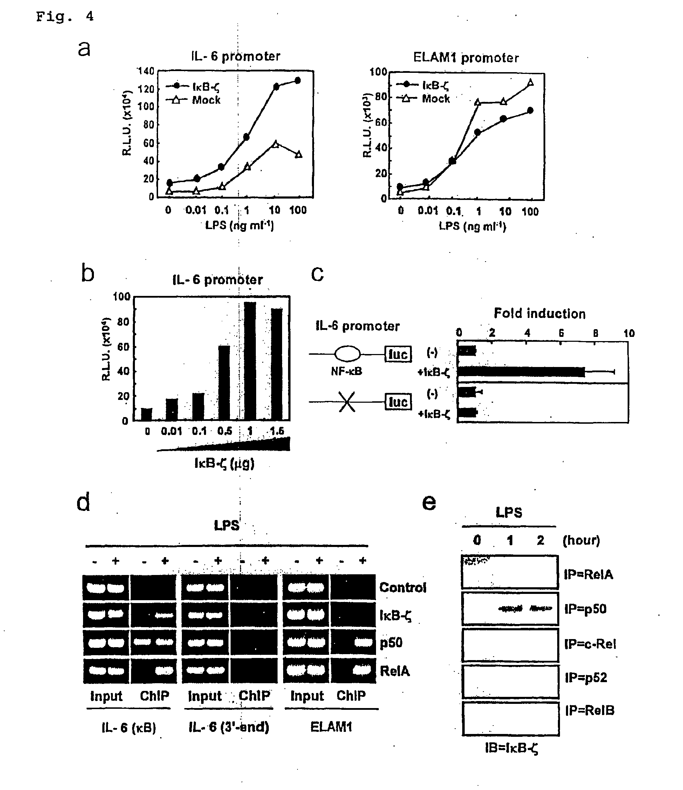

[Fig. 4] It is a figure showing the result of an in vitro analysis of IκB-ζ on the IL-6 promoter. a. RAW 264.7 cells were transiently transfected with luciferase reporter constructs of either mouse IL-6 promoter or ELAM1 promoter together with either a control or the IκB-ζ expression plasmid. Luciferase activities were expressed as fold-increase over the background shown by lysates prepared from untransfected cells. Data are representative of three separate experiments. In all three experiments, cells were stimulated with the indicated concentrations of LPS. b. A certain amount of IκB-ζ expression vector was transiently introduced into untreated RAW 264.7 cells with the indicated amount of IL-6 reporter plasmid. Data are representative of three independent experiments. c. Reporter constructs of either a wild-type or mutant IL-6 promoter were transiently introduced into P19 embryonic canrinoma cells together with either a control or an IκB-ζ expression plasmid. Luciferase activities were normalized in each case of reporter constructs by dividing the increase rate of IκB-ζ-expressed cells over the background shown by lysates by that of mock-expressed cells. d. Chromatin from untreated (-) or LPS-treated (+) (1 µg ml⁻¹ for 3 hours) RAW 264.7 cells were used for ChIp with the indicated antibodies. Precipitated DNA for the IL-6 κB site (left), 3' region of IL-6 gene (center), or ELAM1 promoter (right) was measured by PCR (ChIP). Data are representative of two independent experiments. e. Unstimulated or LPS-stimulated (10 ng ml⁻¹) peritoneal macrophages were immunoprecepitated with the indicated antibodies. The immunoprecipitated lysates were subsequently immunoblotted with α-IκB-ζ.

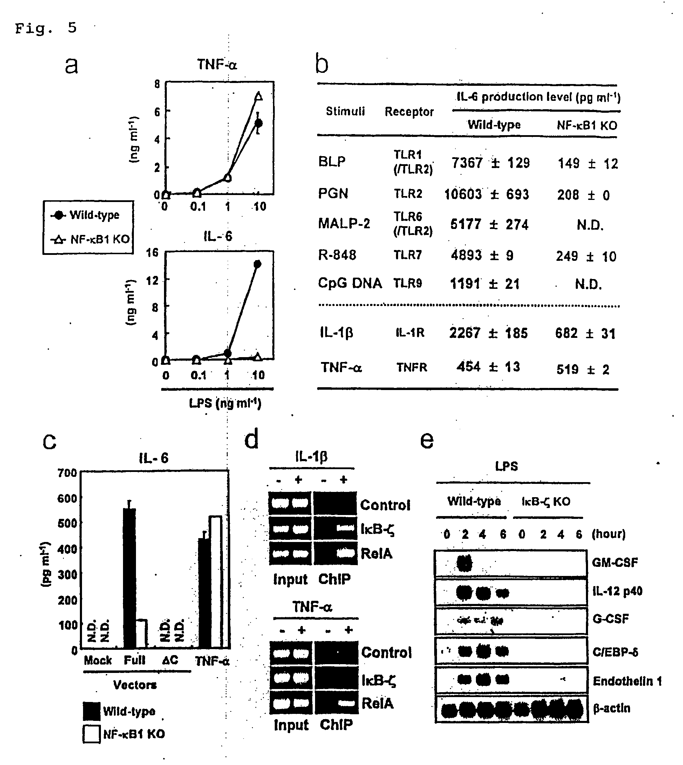

[Fig. 5] It is a figure showing the TLR/IL-1R responses in NF-κB1-deficient mice and microarray analysis result of IκB-ζ-deficient cells. a. Peritoneal macrophages from wild-type and NF-κB1-deficient mice were cultured with 10 ng ml⁻¹ LPS in the presence of 30 ng ml⁻¹ IFN-γ for 24 h. Concentrations of IL-6 and TNF-α in the culture supernatants were measured by ELISA. Indicated values shown in the figure are means ± SD of triplicates. b. Peritoneal macrophages and MEFs from wild-type and NF-κB1-deficient mice were cultured in the coexistence of 100 ng ml⁻¹ BLP, 10 µg ml⁻¹ PGN, 30 ng ml⁻¹ MALP-2, 100 nM R-848, 3 µM CpG DNA, 10 ng ml⁻¹ IL-1β or 10 ng ml⁻¹ TNF-α, in the presence of 30 ng ml⁻¹ IFN-γ for 24h. IL-1β and TNF-α-induced IL-6 production were analyzed by MEFs. Concentrations of IL-6 in the culture supernatants were measured by ELISA. Indicated values are means ± SD of triplicates. c. Full-length IκB-ζ (Full) or deletion-mutant form of IκB-ζ (ΔC) were introduced into wild-type and NF-κB1-deficient MEFs by using retrovirus. Also the same lines of untransfected cells were stimulated with 10 ng ml⁻¹ TNF-α. Concentrations of IL-6 in the culture supernatants were measured by ELISA. Indicated values are means ± SD of triplicates. d. Chromatin from untreated (-), IL-1β-treated (upper, +) (10 ng ml⁻¹ for 3 hours) or TNF-α-treated (lower, +) (10 ng ml⁻¹ for 3 hours) MEFs were used for ChIP with the indicated antibodies shown in the figure. Precipitated DNA for the input (left), or the IL-6κB site (right) was assayed by PCR (ChIP). Data are representative of two independent experiments. e. Peritoneal macrophages from wild-type and NF-κB1-deficient mice were stimulated with 10 ng ml⁻¹ LPS for the indicated periods. Total RNA (5 µg) was extracted and subjected to Northern blot analysis for investigating the expression of the indicated probes.

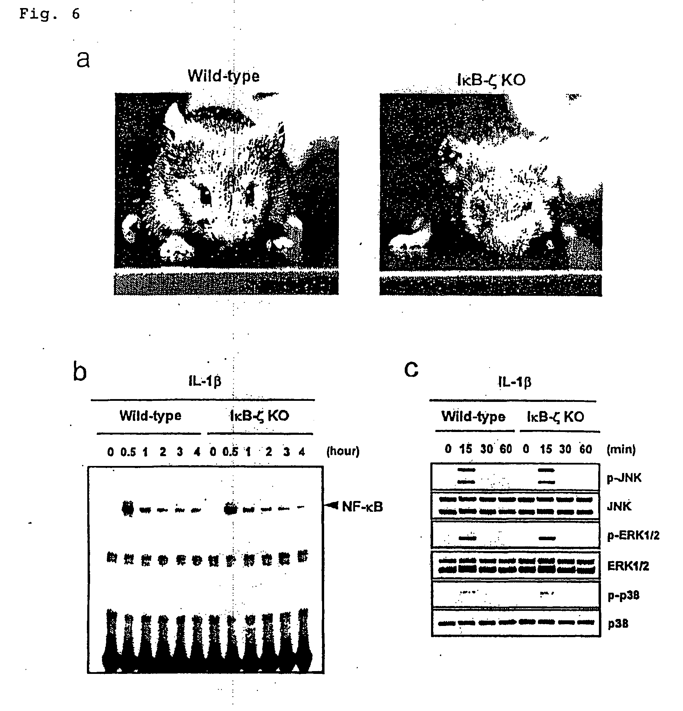

[Fig. 6] It is a figure showing in vivo IL-6 production in IκB-ζ-deficient mice, and IL-1β-induced activation of signaling pathway in IκB-ζ-deficient MEFs. a. It is a figure showing the gross appearance of 16 week-old IκB-ζ-deficient (right) and wild-type mice (left) . b. MEFs were stimulated with 10 ng ml⁻¹ IL-1β for the indicated periods. Nuclear extracts were prepared and NF-κB DNA binding activity was determined by EMSA using an NF-κB-specific probe. c. Activation of JNK, p38, and ERK was also measured by western blotting of cell extracts using anti-phospho-JNK, p38, and ERK.

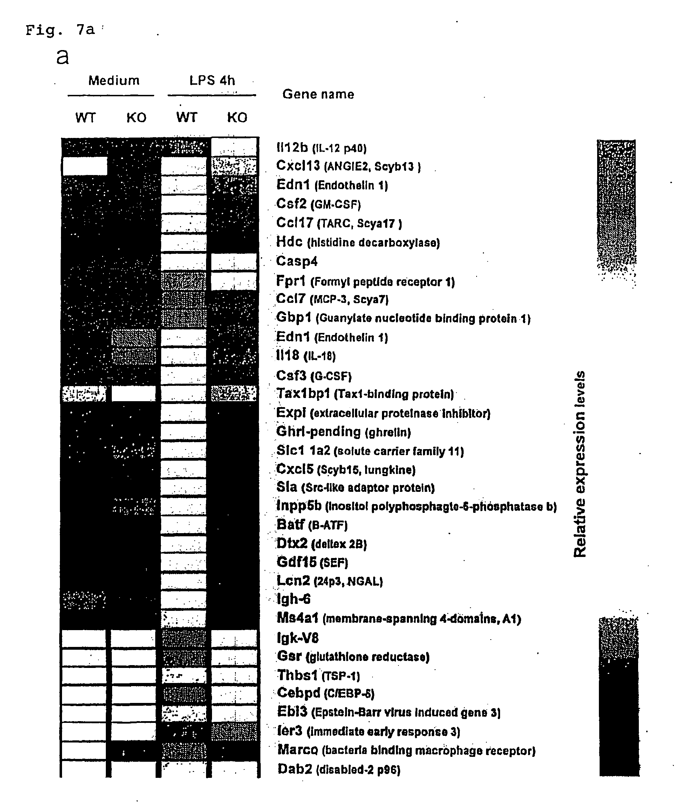

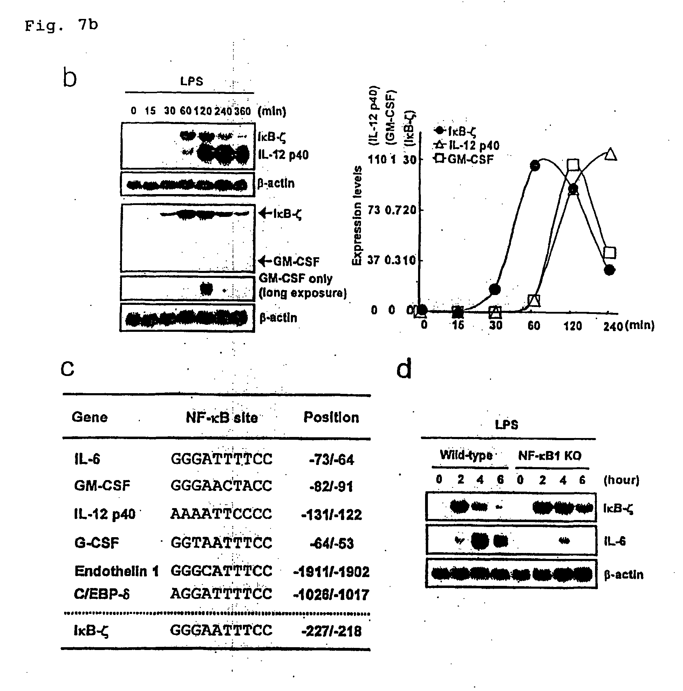

[Fig. 7a] It is a figure showing the results of gene expression analysis of LPS-inducible genes with statistically selected genes. a. It is a figure showing experimental and gene trees obtained from a result of Affimetrix microarray analysis that compared gene expression profiles between wild-type macrophages and IκB-ζ-deficient macrophages in response to 100 ng ml⁻¹ LPS. Genes "expressed" are shown in red and "less expressed" are shown in blue. Relative expression levels are shown in the right column.
[Fig. 7b] It is a figure showing double RNA products indicative of mRNA transcripts derived from IκB-ζ, GM-CSF, and IL-12p40 following LPS simulation of wild-type peritoneal macrophages resolved by electrophoresis. Two-independent experiments with independently derived wild-type cells were quantitated by Phospholmager and mRNA amount was graphed for the indicated genes in arbitrary units (left for IκB-ζ, and IL-12p40, right for GM-CSF) relative to β-actin. c. It is a figure showing NF-κB sites in the promoters/enhancers under investigation. Positions, corresponding to transcription start sites, when mapped are shown. d. Peritoneal macrophages from wild-type and NF-κB1-deficient mice were stimulated with 10 ng ml⁻¹ LPS for the indicated periods. Total RNA (5 µg) was extracted and subjected to Northern blot analysis for investigating the expression of the indicated probes.

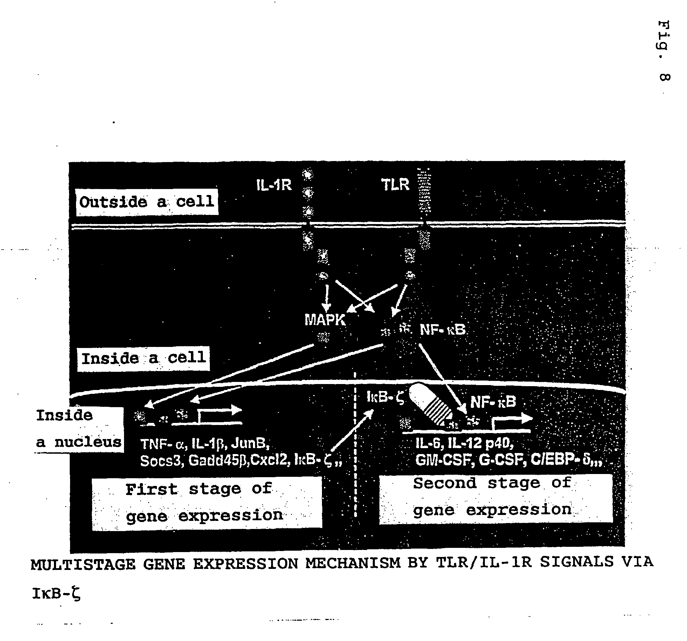

[Fig. 8] It is a figure showing a frame format illustrating a multistage gene expression mechanism by TLR/IL-1R signals via IκB-ζ.

### Best Mode of Carrying Out the Invention

The non-human animal model unresponsive to a TLR ligand and IL-1 of the present invention is not particularly limited as long as it is a non-human animal wherein whole or a part of an endogenous gene of the non-human animal encoding IκB-ζ is inactivated by a gene mutation such as destruction, deletion, or substitution, a function of expressing 1κB-ζ is deleted, a function of expressing IκB-ζ which is expressed in a wild-type is impaired, and a IL-6 production responding to a TLR ligand and IL-1 is impaired. The above-mentioned TLR ligands include: lipopolysaccharide(LPS; TLR4 ligand) which is a glycolipid specific to a cell wall of Gram-negative bacteria; a bacterial lipoprotein (BLP; TLR1/TLR2 ligands); peptidoglycan (PGN; TLR2 ligand) wherein a relatively short peptide chain is bound to a repetitive polysaccharide of N-acetylglucosamine and N-acetylmuramicacid, which are a skeletal structure of a bacterial cell wall; a macrophage-activating lipopeptide (MALP-2; TLR6/TLR2 ligands) derived from bacteria belonging to the genus Mycoplasma; a synthetic compound R-848 (TLR7 ligand) and a DNA having the bacterium-derived unmethylated CpG motif (CpG DNA; TLR9 ligand).

Unresponsiveness mentioned in the present invention means that a responsiveness of a living body, or cells, tissues, or organs constituting the living body against a stimulation with a TLR ligand and IL-1 is lowered or almost lost. Therefore, when used in the present invention, a non-human animal model unresponsive to a TLR ligand and IL-1 refers to a animal beside human such as mouse, rat, or rabbit wherein a responsiveness of living bodies, or cells, tissues, or organs constituting a living body against a stimulation with a TLR ligand and IL-1 is lowered or almost lost. Among them, a non-human animal showing atopic dermatitis-like inflammatory skin disease is preferable. A stimulation with a TLR ligand and IL-1 includes a stimulation in a living body to which a TLR ligand such as LPS or IL-1 is administered, and a stimulation in vitro where TLR ligands such as LPS and IL-1 are allowed to contact with cells separated from a living body.

As a non-human animal of the present invention wherein a function to express IκB-ζ has been lost, a rodent such as an IκB-ζ^{-/-}rat which impairs a function of IκB-ζ gene, as well as an IκB-ζ^{-/-} mouse, can be exemplified. As a non-human animal wherein a function of IκB-ζ gene is deleted, IκB-ζ^{-/-} non-human animals which are born according to Mendel's law are preferable, in terms of that their wild-type littermates, which allow to conduct a precise comparative experiment, are simultaneously obtainable. A method for preparing animals wherein a function of IκB-ζ gene is deleted, will be explained below by taking an IκB-ζ^{-/-} mouse as an example.

A IκB-ζ gene can be obtained by amplifying a mouse gene library by PCR method or the like, and by screening the obtained gene fragment by using a probe derived from mouse IκB-ζ gene. The screened IκB-ζ gene is subcloned using a plasmid vector and the like, and identified by restriction mapping and DNA sequencing. Next, a targeting vector is constructed by replacing whole or a part of a gene encoding IκB-ζ with pMC1neo gene cassette or the like, and introducing a gene such as a diphtheria toxin A fragment (DT-A) gene and a herpes simplex virus thymidine kinase gene (HSV-tK) into 3'-terminal side.

The constructed targeting vector is linearized and introduced into ES cells by a method such as electroporation for homologous recombination, then the ES cells which are homologously recombined by an antibiotic such as G418 and ganciclovir (GANC) are selected among those homologous recombinants. It is preferable to confirm by Southern blotting analysis and so forth whether these selected ES cells are the targeted recombinants. The clones of the confirmed ES cells are microingected into blastocyst of a mouse, and the blastocyst is placed back into the host parent to generate a chimeric mouse. A heterozygout mouse (F1 mouse; IκB-ζ^{+/-}) can be obtained by intercrossing the chimeric mouse with a wild-type mouse, and a IκB-ζ^{-/-} mouse of the present invention can be generated by intercrossing these heterozygote mice. In addition, as a method to confirm whether IκB-ζ is produced in a IκB-ζ^{-/-} mouse, a method comprising the steps of isolating RNA from the mouse obtained by the above-mentioned method and examining the production by Northern blotting or the like, or a method to examine the expression of IκB-ζ in the mouse by Western blotting or the like can be exemplified.

Moreover, whether generated IκB-ζ^{-/-} mouse is unresponsive to a TLR ligand and IL-1 can be confirmed by, for example, allowing to contact LPS with immune cells such as macrophages, mononuclear cells, and dendritic cells of IκB-ζ^{-/-} mouse in vitro or in vivo and measuring a production level of IL-6, IL-12p40, GM-CSF, and the like in the cells. Alternatively, it can be confirmed by observing the level of development of atopic dermatitis-like inflammatory skin disease in aged mice (e.g., 10 weeks-old, preferably 15 weeks-old or older).

A method for screening a substance promoting or inhibiting a response to a TLR ligand or IL-1, or a material containing the same of the present invention is not particularly limited as long as it is a method for measuring/evaluating a IL-6 production level responsive to a TLR ligand or IL-1, or a material containing the same in the non-human animal of the present invention by use of the non-human animal, a test substance, and a TLR ligand or IL-1, or a material containing the same, or a method for measuring/evaluating a IL-6 production level responsive to a TLR ligand or IL-1, or a material containing the same in immune cells such as macrophages, splenocytes, or dendritic cells derived from a non-human animal of the present invention by use of the immune cells, a test substance, and a TLR ligand or IL-1, or a material containing the same. Embodiments of the methods for screening of the present invention will be explained by referring to the following examples.

Exemplified methods for screening include: a screening method in which immune cells such as macrophages obtainable from the non-human animal model of the present invention, a test substance, and a TLR ligand or IL-1, or a material containing the same are cultured together, followed by measuring/evaluating a IL-6 production level in the immune cells; a screening method comprising the steps of administrating a test substance to the non-human animal model of the present invention in advance, culturing immune cells obtainable from the non-human animal in the presence of a TLR ligand or IL-1, or a material containing the same, and measuring/evaluating a IL-6 production level in the immune cells; a screening method comprising the steps of administrating a TLR ligand or IL-1, or a material containing the same to the non-human animal model of the present invention in advance, culturing immune cells obtainable from the non-human animal in the presence of a test substance, and measuring/evaluating a IL-6 production level in the immune cells; a screening method comprising the steps of administrating a TLR ligand or IL-1, or a material containing the same, and a test substance simultaneously or either one first to the non-human animal model of the present invention, and measuring/evaluating a IL-6 production level in the immune cells obtainable from the non-human animal.

A method for screening a prophylactic/therapeutic agent for atopic dermatitis-like inflammatory skin disease of the present invention is not particularly limited as long as it is a method in which a test substance is administrated to the non-human animal of the present invention, and the level of atopic dermatitis-like inflammatory skin disease in the non-human animal is measured/evaluated. A test substance can be administrated either before or after a development of atopic dermatitis-like inflammatory skin disease. A test substance is useful as a prophylactic agent in a case where the symptom is improved by the administration thereof before development, and useful as a therapeutic agent in a case where the symptom is improved by the administration thereof after development. A method for measuring/evaluating a level of atopic dermatitis-like inflammatory skin disease includes a method for observing visually or the like the presence or absence of palpebral edema; edema of lips and face; eczema and alopecia of face and neck, forelimb flexion parts, and thorax; forelimb pruritic behavior; exudation, blood crust, and erosion of the face skin caused by peeling.

The present invention will be explained more specifically by referring to examples in the followings, however the technical scope of the invention is not limited thereto.

### Example 1

### [Specific induction of IκB-ζ with TLR/IL-1R stimulation]

Whether IκB-ζ would be induced in response to other TLR ligands was examined based on the fact that stimulation with IL-1 or LPS induces IκB-ζ in macrophage cell lines and fibroblasts, and that IL-1R and the receptor for LPS, TLR4, share the intracellular signaling pathways. In addition to IL-1 and LPS, IκB-ζ mRNA was strongly induced in peritoneal macrophages or lung-derived normal cells on stimulation with peptidoglycan (PGN)(TLR2 ligand), bacterial lipoprotein (BLP)(TLR1/TLR2 ligands), flagellin (TLR5 ligand), MALP-2 (TLR6/TLR2 ligands), R848 (TLR7 ligand), and CpG DNA (TLR9), but no induction was observed with TNF-α (Fig. 1a). On the other hand, other IκB family members, IκB-α and Bcl-3, were induced in response to TNF-α as well as a TLR ligand and IL-1. Taken together, unlike IκB-α and Bcl-3, the IκB-ζ induction is specifically detected on the TLR/IL-1R stimulation.

Next, whether the induction of IκB-ζ was regulated by the MyD88-dependent or -independent pathway was examined. In embryonic fibroblasts (MEFs) from MyD88-deficient mice, the induction of IκB-ζ in response to IL-1 and LPS was completely abolished (Fig. 1b). Thus, IκB-ζ was found to be an inducible protein in the MyD88-dependent pathways of the TLR/IL-1R signaling.

### Example 2

### [IκB-ζ gene knockout mouse]

To elucidate the in vivo role of IκB-ζ in the TLR/IL-1R response, IκB-ζ-deficient mice were generated by targeted gene disruption. A targeting vector in which two exons encoding the central portion of IκB-ζ were replaced with the neomycin resistance (neoR) gene was constructed (Fig. 2a). Homologous recombination in embryonic stem (ES) cells was confirmed by Southern blot analysis, and correctly targeted ES cells were used to generate mice with the mutated allele (Fig. 2b). Homozygous mice were born according to Mendel's law. Expression of IκB-ζ mRNA in the mutant mice was investigated by Northern blot analysis. LPS stimulated the IκB-ζ induction in wild-type cells, however, no IκB-ζ mRNA was detected in IκB-ζ-deficient cells (Fig. 2c). Immunoblot analysis further confirmed that disruption of the IκB-ζ gene abolished the expression of IκB-ζ protein (Fig. 2d). IκB-ζ-deficient mice had normal composition of B and T lymphocytes. However, IκB-ζ-deficient splenocytes showed defective proliferation in response to LPS, but rewponsiveness to α-CD40, IL-4, and α-IgM was not impaired (Figs. 2e and 2f), suggesting that the TLR4 response is impaired in IκB-ζ-deficient cells.

Moreover, all of aged IκB-ζ-deficient mice showed atopic dermatitis-like inflammatory skin disease (Fig. 6a). Details of analysis for the skin phenotype are described in numerous literatures.

### Example 3

### [Immune response and IκB-ζ induction kinetics in IκB-ζ-deficient cells]

Next, the LPS-induced production of inflammation-associated molecules in peritoneal macrophages was analyzed. Wild-type macrophages produced TNF-α and nitric oxide (NO) in response to LPS in the presence of IFN-γ (Fig. 3a). Although LPS-induced TNF-α and NO production were normal, the IL-6 production was severely reduced in IκB-ζ-deficient mice. Moreover, IL-6 production stimulated with other TLR ligands such as CpG DNA, R-848 , PGN, MALP-2, and BLP was also profoundly inhibited in IκB-ζ-deficient cells (Figs. 3b, 3c, and 3d). In addition, IκB-ζ-deficient cells exhibited defective IL-6 production in response to IL-1. However, TNF-α-induced IL-6 production was found to be comparable in both wild-type and IκB-ζ-deficient cells (Fig. 3e). Next, the IL-6 mRNA expression in response to LPS was investigated. LPS stimulated the IL-6 mRNA induction in wild-type cells. In sharp contrast, the IL-6 mRNA levels were dramatically reduced in IκB-ζ-deficient cells (Fig. 3f), demonstrating that the decreased IL-6 production level in IκB-ζ-deficient cells may reflect the impaired mRNA induction.

As described above, it is known that the TLR/IL-1R-mediated MyD88-dependent pathways associated with the inflammatory cytokine production activate NF-κB and mitogen-activated protein (MAP) kinases. However, the activation of NF-κB and MAP kinases in response to IL-1 was similar in both wild-type and IκB-ζ-deficient cells (Figs. 6b and 6c). Next, the full-length IκB-ζ or a deletion mutant form of IκB-ζ was transfected in medium- or IL-1-treated IκB-ζ-deficient MEFs. Under IL-1-stimulated conditions, the full-length of IκB-ζ, rescued the defective IL-6 production in IκB-ζ-deficient cells while rescue was observed for the deletion mutant form of IκB-ζ (Fig. 3g), suggesting that IκB-ζ expression as well as the activation of NF-κB and MAP kinases is required for the TLR/IL-1-mediated IL-6 production.

As described above, both IκB-ζ and IL-6 have been reported as inducible genes in response to a TLR ligand and IL-1. Accordingly, to measure kinetics for both of genes, time-course Northern blot analysis using peritoneal macrophages was performed. Upon LPS stimulation, the IκB-ζ induction was observed 30 min after LPS stimulation and the induction reached its maximum level 120 min after. On the other hand, IL-6 mRNA was induced at later time points than that of IκB-ζ or TNF-α (Fig. 3h). Taken these results together, it is suggested that the TLR/IL-1R-mediated IL-6 gene expression requires a preceding induction of IκB-ζ. Given that IκB-ζ is also an inducible protein in signaling pathways via TLR/IL-1R, the TLR/IL-1R-mediated pro-inflammatory IL-6 production is thought to be controlled in at least a two-step fashion.

### Example 4

### [In vitro analysis of IκB-ζ on IL-6 promoter]

To investigate whether IκB-ζ promotes the ligand-induced activation of the IL-6 promoter, luciferase reporter plasmids having a promoter of either IL-6 or ELAM1 gene were introduced together with control or IκB-ζ expression vectors in RAW 264.7 cells. LPS stimulated the activation of both IL-6 and ELAM1 promoters in a dose-dependent manner. It was found that, when IκB-ζ, is overexpressed, IL-6 promoter activities were further enhanced in RAW 264 . 7 cells while the tendency was not observed for ELAM1 (Fig. 4a). Since the enhancement was observed in unstimulated cells, whether etopic expression of IκB-ζ had positive effects on the activities of IL-6 promoter in nonstimulated RAW 264.7 cells was examined. By increasing the induction level of IκB-ζ, it was observed that the basal activities of IL-6 promoter were also upregulated in nonstimulated cells (Fig. 4b).

Next, examination was made to determine which portions of the IL-6 promoter were associated with the enhancement of IκB-ζ overexpression. Since the NF-κB binding site in the IL-6 promoter has been shown to play important roles in the activation, P19 embryonic carcinoma cells were transfected with either a wild-type reporter or mutant reporter in which the NF-κB binding site was disrupted (Proc Natl Acad Sci USA. 90, 10193-10197 (1993)). Ectopic expression of IκB-ζ activated the wild-type reporter. In contrast, when the mutant reporter was introduced, the effect of IκB-ζ overexpression was completely abolished (Fig. 4c).

To examine the specific involvement of IκB-ζ in κB site in the IL-6 promoter directly, chromatin immunoprecipitation assay (ChIP) using antibodies including α-IκB-ζ, α-NF-κBp50, α-NF-κB, RelA, and control antibodies was performed. The κB sites from the IL-6 and the ELAM1 promoter, but not the 3' terminal of the IL-6 gene, were detected when LPS-stimulated cells were immunoprecipitated with α-p50 and α-RelA. Especially, the p50 subunit was readily detected in the IL-6 κB site in unstimulated cells as previously reported (Mol Cell. 9,625-636 (2002)). By sharp contrast, the IL-6 κB site, but neither the ELAM1 κB site nor the 3' terminal of the IL-6 gene, was co-immunoprecipitated with α-IκB-ζ in LPS-stimulated cells (Fig. 4d), demonstrating the selective involvement of IκB-ζ in the IL-6 promoter.

Finally, association of IκB-ζ with NF-κB family members was investigated. As a result of immunoprecipitation analysis, interaction of IκB-ζ proteins with p50 was observed, but with neither Re1A, RelB, c-Rel, nor the p52 subunit (Fig. 4e). These findings indicate that positive effects by IκB-ζ may be exerted through the association with the p50 subunit.

### Example 5

### [TLR/IL-1R responses in NF-κB1-deficient mice and microarray analysis of IκB-ζ-deficient cells]

To confirm the above-described hypothesis by genetic means, peritoneal macrophages and MEFs were obtained from NF-κB1/p50-deficient mice and LPS-induced cytokine production was analyzed. As previously reported, LPS-induced TNF-α production in NF-κB1-deficient macrophages was equivalent to that in wild-type cells in the presence of IFN-γ. On the other hand, NF-κB1-deficient cells showed defective IL-6 production in response to LPS (Cell. 80, 321-330 (1995), Proc Natl Acad Sci USA. 97,12705-12710 (2000), and Fig. 5a). Additionally, IL-1- and other TLR ligands-induced IL-6 production were severely impaired in NF-κB1-deficient cells (Fig. 5b). Consequently, the TLR/IL-1R-mediated responses in NF-κB1-deficient mice were reminiscent of those in IκB-ζ-deficient mice.

Furthermore, it was tested whether ectopic expression of IκB-ζ induces IL-6 production in NF-κB1-deficient cells. Compared with wild-type cells, IκB-ζ-mediated IL-6 production was reduced in NF-κB1-deficient MEFs, indicating that NF-κB1 plays an important role in the exertion of the effect of IκB-ζ (Fig. 5c). Both IL-1 stimulation and TNF-α stimulation lead to activation of similar sets of signaling molecules such as NF-κB and MAP kinases, culminating in expression of IL-6. However, signaling via an IL-1/TLR ligand specifically recruited IκB-ζ to the IL-6 promoter, but such a recruitment was not observed in signaling via TNF-α, (Fig. 5d), presumably accounting for the difference between TLR/IL-1R and TNFR responsiveness.

Next, other LPS-inducible genes regulated by IκB-ζ were searched using microarray analysis. Many of LPS-inducible genes were significantly affected by the IκB-ζ deficiency (Fig. 7a). Several of the genes were subsequently tested by Northern blot analysis to confirm the accuracy. Among them, LPS-induced expression of GM-CSF, IL-12 p40, G-CSF, C/EBP-δ, and Endothelin 1 was decreased in IκB-ζ-deficient macrophages (Fig. 5e), showing that IκB-ζ regulates expression of the genes in addition to IL-6. Indeed, results of time-course Northern blot analysis showed that the kinetics for induction of these genes in response to LPS were similar to that of IL-6 rather than TNF-α (Figs. 3h and 7b), further supporting the model of at least a two-step regulation of the above-mentioned genes in TLR/IL-1R signaling pathways.

Finally, it was examined whether the IκB-ζ requirement correlates with the sequence of the κB sites from the tested genes. However, distinct κB sites were contained in the promoter and any common features in the relevant κB site sequences could not be discerned (Fig. 7c), assuming that the capability of IκB-ζ as well as NF-κB to function on a particular promoter is probably determined not only by DNA-protein interactions at the κB site but also by other transcription factors or co-activators that bound or interact with the neighboring base sequence (EMBO J. 22, 5530-5539 (2003)). Moreover, regardless of the κB site in the IκB-ζ promoter showing highest homology with the κB site in the IL-6 promoter among them (Fig. 7c), LPS-induced IκB-ζ mRNA expression was normally observed in NF-κB1-deficient cells (Fig. 7d), indicating that the regulation of IκB-ζ itself in response to LPS is different from that of IL-6.

The present invention provided genetic evidence that IκB-ζ plays an important role in the TLR/IL-1R-mediated immune response. Especially, in the case of TLR/IL-1R-mediated IL-6 production, the preceding IκB-ζ expression as well as the activation of NF-κB and MAP kinases may be required for the appropriate production. Since IκB-ζ itself is an inducible protein, the TLR/IL-1R-mediated IL-6 expression may be regulated in a two-step mechanism. In addition, microarray analysis results showed that IκB-ζ might control other LPS-inducible genes than IL-6. It is expected that further analysis that clarifies the molecular basis of IκB-ζ-dependent gene expression will provide a new insight in the TLR/IL-1R-mediated MyD88-dependent immune responses.

### Example 6

### [Method]

### (1) Generation of IκB-ζ-deficient mice

Genomic DNA containing the IκB-ζ gene was isolated from 129/SV mouse genomic library and characterized by restriction mapping and sequencing analysis. The targeting vector was constructed by replacing a 2.0-kb fragment encoding the central portion of IκB-ζ with a neoR cassette, and a herpes simplex virus thymidine kinase transcribed by the PGK promoter in the genomic fragment was inserted for negative selection (Fig. 2a). The targeting vector was transfected into embryonic stem cells (E14.1). G418 and gancyclovir doubly resistant colonies were selected and screened by PCR and Southern blotting. Two independent homologous recombinants were micro-injected into C57BL/6 female mice and heterozygous F1 progenies were intercrossed to obtain IκB-ζ-deficient mice. Mice from these independent clones showed identical phenotype. IκB-ζ-deficient mice and their wild-type littermates at the age of 6 to 8 weeks were used in the experiments of the present invention. All animal experiments were conducted with the approval of the Animal Research Committee of the Research Institute for Microbial Diseases (Osaka University, Osaka, Japan).

### (2) Reagents and mice

LPS derived from Salmonella Minnesota Re595, and PGN from S. aureus were purchased from Sigma and Fluka, respectively. MALP-2 and CpG oligodeoxynucleotides were prepared as described previously (J Immunol. 164, 554-557 (2000), Nature. 408, 740-745 (2000)). Flagellin and R-848 were provided by Dr. A. Adrem and Dr. H. Tomizawa, respectively. Polyclonal antibody against IκB-ζ was obtained by immunizing a rabbit with the bacterially expressed C-terminal portion of mouse IκB-ζ protein. Antibodies against each of phosphorylated ERK, JNK, and p38 were purchased from Cell Signaling. Antibodies against each of ERK, JNK, p38, RelA, p50, c-Rel, and C/EBP-β were obtained from Santa Cruz. Recombinant TNF-α and IL-1β were obtained from Genzyme. NF-κB1/p50- or MyD88-deficient mice were generated in accordance with the method described previously (Cell.80, 321-330 (1995), Proc Natl Acad Sci USA. 89, 1473-1476 (1992).

### (3) Measurement of pro-inflammatory cytokine concentrations and NO

Thioglycollate-elicited peritoneal macrophages or MEFs were cultured in 96-well plates (5 × 10⁴ cells per well for macrophages, 1 × 10⁴ cells per well for MEFs) with the indicated concentrations of the indicated ligands. Concentrations of TNF-α and IL-6 in the culture supernatants were measured by ELISA according to manufacturer's instructions (Genzyme for TNF-α, R&D for IL-6). Concentrations of NO were measured by Griess method according to manufacturers' instruction (DOJINDO).

### (4) Electrophoretic mobility shift assay

MEFs (1 × 10⁶) were stimulated with 10 ng ml⁻¹ IL-1β for the indicated period. Nuclear extracts were purified from cells and incubated with a specific probe for NF-κB DNA binding site, electrophoresed, and visualized by autoradiography, according to the method described previously (Nature. 408, 740-745 (2000)).

### (5) Plasmids and retroviral transfection

The reporter plasmids constituted of 5' flankin region (-1240/+40) of the mouse IL-6 gene, were used in Figs. 4a and 4b. The reporter plasmids used in Fig. 4c were as listed in Proc Natl Acad Sci USA. 90, 10193-10197 (1993); Proc Natl Acad Sci USA. 89, 1473-1476 (1992). The ELAM1 promoter-derived luciferase reporter plasmid was provided from D.T.Golenbock. The full-length (Full) or deletion mutant form of IκB-ζ lacking the C-terminal portion (ΔC, J Biol Chem. 276, 27657-27662 (2001) ) were cloned into pMRX retroviral vector as described previously (J Biol Chem. 278,36005-36012 (2003)). Plat-E packaging cells were kindly provided from Dr. T. Kitamura (Gene Ther. 7, 1063-1066(2000)). Describing briefly, for producing retroviruses, Plat-E cells were transfected using Lipofectamine 2000 reagent (Invitrogen), and the culture supernatants of Plat-E cells containing retroviruses were collected 60 h after transfection. MEFs were infected in the presence of 4 µg/ml of polybrene for 6 h, and subsequently analyzed 48 h after infection.

### (6) Luciferase reporter assay

Reporter plasmids were transiently co-transfected into RAW 264.7 and P19 cells with either control or the 1κB-ζ expression vectors by using SUPERFECT transfection reagent (QIAGEN). Luciferase activities of total cell lysates were measured using dual-luciferase reporter assay system (Promega, Maiden, WI) according to the method described previously (28, 29) (Nat Immunol. 3, 392-398 (2002); Nat Immunol. 2, 835-841 (2001)).

### (7) Chromatin immunoprecipitation (ChIP assay)

ChIP assay was performed essentially according to a protocol of the manual (Upstate Biotechnology). In brief, 2 × 10⁶ RAW 264.7 cells or 5 × 10⁵ MEFs were stimulated with LPS (1 µg ml⁻¹, 3 h), IL-1β (10 ng ml⁻¹, 3 h) or TNF-α (10 ng ml⁻¹, 3 h), respectively, and then fixed with formaldehyde for 60 min at 37°C. The cells were lysed, sheared by sonication, and incubated overnight in the coexistence of a specific antibody, followed by an incubation with protein A-agarose saturated with salmon sperm DNA (Upstate Biotechnology). Precipitated DNAs were analyzed by quantitative PCR (35-40 cycles) using the following primers.

Primers used for quantitative PCR; 5'-CGATGCTAAACGACGTCACATTGTGCA-3' (SEQ ID No: 1) and 5'-CTCCAGAGCAGAATGAGCTACAGACAT-3' (SEQ ID No: 2) for κB site in the IL-6 promoter, 5'-GCAGATGGACTTAGCTCGTCTCATTCA-3' (SEQ ID No: 3) and 5' -CCACTCCTTCTGTGACTCCAGCTTATC-3' (SEQ ID No: 4) for a 3' gene segment in the IL-6 promoter, 5'-GATGCAGTTGAGAATTTCCTCTTAGCC-3' (SEQ ID No: 5) and 5'-TGGAATAGTTGTTCTGGCGTTGGATCC-3' (SEQ ID No: 6) for κB site in the ELAM1 promoter.

### (8) Western blot analysis and immunoprecipitation

Peritoneal macrophages and MEFs were stimulated with the indicated ligands for the indicated periods. The cells were then lysed in a lysis buffer containing 1.0% Nonidet-P40, 150 mM NaCl, 20 mM Tris-Cl (pH 7.5), 5 mM EDTA and protein inhibitor (Roche). The cell lysates were dissolved by SDS-PAGE and transferred on a PVDF membrances. For immunoprecipitation, cell lysates were precleared with Protein G-Sepharose™ (Amersham Pharmacia Biotech) for 2 h and then incubated with Protein G-Sepharose™ containing 1.0 µg of the indicated antibodies for 12 h with stirring at 4°C. The immunoprecipitants were washed four times with lysis buffer, eluted by boiling with Laemmli sample buffer, and subjected to Western blotting using α- IκB-ζ, as described above.

### (9) Gene Chip analysis

Microarray analysis (Affimetrix) using wild-type and IκB-ζ-deficient peritoneal macrophages in response to LPS was performed according to the method described in Blood. 102 4123-4129 (2003). GeneSpring6.0 (Silicon Genetics) software was used to generate a color image of gene expression.

### Industrial Applicability

The present invention introduces a new notion of "a two-stages (multistage) expression control in TRL and IL-1R signaling response" , that is, the notion wherein a further step to preliminarily induce IκB-ζ expression is essential (see Fig. 8), instead of a conventional notion with a one-stage expression control wherein a gene expression in TLR/IL-1R signaling pathways is thought to have only one step which is the NF-κB and AP-1 activation. Whereby a multi-step gene expression control mechanism with TLR/IL-1R can be elucidated. Moreover, as IκB-ζ-deficient mice show atopic dermatitis-like inflammatory skin disease, the future pathological analysis of atopic dermatitis-like inflammatory skin disease in IκB-ζ-deficient mice and the detailed analysis of gene expression control are expected to create, through convergence thereof, a new treatment strategy for intractable diseases whose causes have not been known.

## Claims

1. A non-human animal model unresponsive to a TLR ligand and IL-1, wherein whole or a part of an endogenous gene of the non-human animal encoding IκB-ζ is inactivated by a gene mutation such as destruction, deletion, or substitution, a function of expressing IκB-ζ is deleted, and a IL-6 production responding to a TLR ligand and IL-1 is impaired.

2. The non-human animal model unresponsive to a TLR ligand and IL-1 according to claim 1, wherein the TLR ligand is LPS, BLP, PGN, MALP-2, R-848, or CpG DNA.

3. The non-human animal model unresponsive to a TLR ligand and IL-1 according to claim 1 or 2, wherein the non-human animal model shows an atopic dermatitis-like inflammatory skin disease.

4. The non-human animal model unresponsive to a TLR ligand and IL-1 according to any one of claims 1 to 3, wherein the non-human animal is a mouse.

5. A method for screening a substance promoting or suppressing a response to a TLR ligand or IL-1, or a material containing the same, wherein the non-human animal according to any one of claims 1 to 4, a test substance, and a TLR ligand or IL-1, or a material containing the same, are used to measure/evaluate an IL-6 production level responding to a TLR ligand or IL-1, or a material containing the same, in the non-human animal.

6. A method for screening a substance promoting or suppressing a response to a TLR ligand or IL-1, or a material containing the same, wherein an immune cell derived from the non-human animal according to any one of claims 1 to 4, a test substance, and a TLR ligand or IL-1, or a material containing the same, are used to measure/evaluate an IL-6 production level responsive to a TLR ligand or IL-1, or a material containing the same in the immune cell.

7. A method for screening a prophylactic/therapeutic agent for atopic dermatitis-like inflammatory skin disease, wherein a test substance is administrated to the non-human animal according to any one of claims 1 to 4 and a level of atopic dermatitis-like inflammatory skin disease in the non-human animal is measured/evaluated.
